# EUROPEAN PATENT SPECIFICATION

(11) **EP 0 727 485 B1**
(45) Date of publication and mention of the grant of the patent: **17.09.2003**
(21) Application number: 96200401.6
(22) Date of filing: 16.02.1996
(51) Int. Cl.: C12N 9/24, C12N 9/90, C12P 19/24, C12P 19/14, A61K 31/70, A61K 7/00, A23L 1/105, A23L 1/236, C11D 3/22

(54) **Method for conversion of a starch material, and enzyme composition suitable therefor**
Verfahren zur Umwandlung von stärkeartigem Material und dafür geeignete enzymatische Zusammensetzung
Méthode pour la conversion d'un matériel amidoné et composition enzymatique adaptée

(30) Priority: 16.02.1995 NL 9500292
(43) Date of publication of application: 21.08.1996
(73) Proprietor: Nederlandse Organisatie voor toegepast-natuurwetenschappelijk onderzoek TNO, 2628 VK Delft (NL)
(72) Inventor: Binnema, Doede Jacob, 9721 TX Groningen (NL); Wijbenga, Dirk-Jan, 9301 WZ Roden (NL); Klip, Gerhard Ronald, 9714 GR Groningen (NL); van Hoogmoed, Christianus Gerhardus, 9737 NS Groningen (NL)
(74) Representative: Smulders, Theodorus A.H.J., Ir.

(56) References cited:
- EP-A- 0 332 027
- EP-A- 0 499 434
- EP-A- 0 606 753
- EP-A- 0 628 630
- EP-A- 0 688 866
- GB-A- 976 088
- BIOTECH. FORUM EUROPE, vol. 8, no. 4, 1991, pages 201-203, XP002003660 LAMA ET AL.: "Thermostable amylolytic activity from Sulfolobus solfataricus"
- DATABASE WPI Week 3285 Derwent Publications Ltd., London, GB; AN 193629 XP002003661 & JP-A-60 120 984 (IKEDA TOKA KOGYO) , 28 June 1985

## Description

### FIELD OF THE INVENTION

The invention lies in the field of starch technology and more particularly concerns methods for converting starch utilizing enzymes. Such methods are generally aimed at the preparation of derivatives of starch which have a modified degree of polymerization.

The invention relates in particular to an enzymatic method for the preparation of starch derivatives having a modified degree of polymerization with respect to the starting starch, with the non-reducing character of the starting starch being substantially maintained.

### BACKGROUND OF THE INVENTION

On the basis of existing knowledge in the state of the art, it is possible, by converting starch into cyclomaltodextrins using cyclomaltodextrin glucanotransferases (EC 2.4.1.19), to obtain products in which the non-reducing character of the starting material has been maintained. The cyclic products formed, however, have different properties, such as a reduced solubility and capacity to complex, than non-cyclic glucans.

The formation of non-cyclic non-reducing glucans has heretofore been found possible by converting starch hydrolysates with a reducing character at temperatures below 60°C into preparations with a non-reducing character. For the formation of non-reducing saccharides, thermolabile enzymes from mesophilic microorganisms are used, as described in European patent publication EP 0 606 753 A2. One of the enzymes is a non-reducing saccharide-forming enzyme, which catalyzes the formation of non-reducing saccharides ending with a trehalose structure. Enzymes of this type will herein be designated as non-reducing glucan (NRG) forming enzymes.

The method according to EP 0 606 753 A2 starts from a starch syrup with a reduced degree of polymerization, which has been obtained by hydrolysis of gelatinized and liquefied starch at temperatures above 60°C according to generally known methods. In this syrup preparation, the reducing character of the starch increases, as does the formation of colored components which are a result of Maillard reactions. The undesired presence of such reaction products, formed by the reaction of aldoses with amino groups, means that in the production of the syrups it is preferred to use starting material that is free from proteins, peptides and amino acids, and also that Maillard products and material with still free amino groups, present in the syrups obtained, have to be removed by generally known methods to prevent further Maillardation during drying.

A method in which starch, optionally directly in a one-step process, can be converted into non-reducing products with a modified degree of polymerization would therefore constitute an important improvement.

It might be tried to realize such a direct conversion of starch into non-reducing products by combining the thermolabile enzymes according to EP 0 606 753 A2, which are only active at temperatures lower than 60°C, with hydrolytic enzymes capable of decomposing starch below the gelatinizing temperature. Amylases capable of doing this are for instance described in an article by Wijbenga et al. (Appl. Microbiol. Biotechnol. 35 (1991) 180-184). However, the most important reaction product here is glucose, which cannot serve as starting material for NRG-forming enzymes from patent specification EP 0 606 753 A2.

A different approach might consist in decomposition with thermostable enzymes active above 60°C, enabling work above the gelatinizing temperature of starch. Hydrolytic enzymes, such as α-, β- and glucoamylases, which are capable of doing so, are commercially available and find extensive application in the industry.

Advantages of conducting such a process at higher temperatures are: reduced chance of microbial contamination, increased reaction rate and a lower viscosity enabling high substrate concentrations. The combination of such thermostable hydrolases with an NRG-forming enzyme (NRGFE) thus has evident advantages. For such a method, however, an NRGFE with thermostable properties is indispensable. Such enzymes are not known, nor have any been found in the extensive screening of microorganisms described in EP 0 606 753 A2.

The above means that according to the present state of the art it is impossible to convert starch directly, i.e. in a one-step process, into non-reducing glucans.

Thermostable enzymes might be obtained by modification at a protein or DNA level of enzymes active at lower temperatures. Alternatively, they might be isolated from nature, in particular from (not necessarily thermophilic) organisms, which may belong to the domains of the *Archaea, Bacteria* and *Eukarya*.

Surprisingly, it has been established that thermostable enzymes with which such a method can be carried out can indeed be isolated from microorganisms belonging to the domain of the *Archaea*. Specifically, it has been found that a thermophilic archaeon belonging to the genus *Sulfolobus,* in particular *Sulfolobus solfataricus* DSM-1617, produces an enzyme which possesses the necessary properties for its use in a method that can be carried out above 60°C.

### SUMMARY OF THE INVENTION

The invention relates to thermostable enzyme preparations comprising an NRG-forming entity that is catalytically active at temperatures higher than 60°C, so that reactions above the gelatinizing temperature of starch are possible.

The invention further relates to a method for converting starch, utilising a combination of thermostable enzymes including an NRG-forming entity, into products having a modified degree of polymerization while the non-reducing character of the starch preparation is maintained.

As will be explained in more detail, the use of thermostable NRG-forming enzymes, in combination with thermostable starch-decomposing hydrolases, such as amylases, isoamylases, (amylo)pullulanases as well as with transferases, enables the direct production from starch of non-cyclic glucans of different chain length, with the non-reducing character of the starting material being maintained.

As starting material in the method according to the invention, a starch material is used. The term starch material here denotes native starch and products with a different degree of polymerization derived therefrom. The starch material to be used can be of various origin, such as potato starch, maize starch, wheat starch, tapioca starch, pea starch, waxy-maize starch and other amylose-free or low-amylose (hence amylopectin-rich) starches, and high-amylose starch. It is also possible to use a modified starch which has been obtained by chemical, enzymatic and/or physical modification of native starch. The term starch material as used here also encompasses such modified starch products.

### DETAILED DESCRIPTION OF THE INVENTION

The invention comprises a method for conversion of a starch material, characterized in that the conversion takes place utilizing a thermostable non-reducing glucan-forming enzyme activity (NRGFE). According to the invention, the conversion preferably occurs at 60-120°C, preferably 70-80°C, and at pH 4-9, preferably pH 5-7.

NRG-forming entity or enzyme activity is intended to refer in particular to enzymes capable of catalyzing the reaction Gₙ --> Gₙ₋₂T, wherein G represents a glucosyl residue, T a trehalosyl unit, and n an integer.

The thermostable NRGFE to be used according to the invention preferably originates from microorganisms, more specifically from *Sulfolobus solfataricus* DSM 1617 or a microorganism descending or derived therefrom. 'Originating from' and 'derived from' are herein intended only to denote origin, and are specifically not intended as a limitation to 'produced by'. Thus the invention comprises the possibility that the enzyme is produced by a recombinant host different from the organism that produces the enzyme by nature.

Although modified and derivatized starch products can function as starting material, as has already been indicated above, it is preferred to use a native starch as starting starch material.

According to one embodiment of the invention, the starch material is depolymerized under simultaneous formation of non-reducing glucans. The depolymerization is preferably carried out with thermostable glucanases, such as in particular thermostable α-amylases, isoamylases, pullulanases, amylopullulanases or combinations thereof. Simultaneously with the depolymerization, non-reducing glucans are formed as a result of the catalytic action of the NRGFE. During the process operation and during subsequent processes, the formation of Maillard products is suppressed by virtue of the permanent absence of reducing terminal ends.

According to another embodiment of the invention, trehalose is formed from the non-reducing glucans by using suitable glucanases, in particular enzymes such as amylases, glucosidases, amyloglucosidases or combinations thereof. An enzyme useful for this purpose can for instance be found in *Sulfolobus solfataricus*. Useful enzymes for the release of trehalose from the NRG formed are described, for instance, in EP 0 628 630 A2.

According to yet another embodiment of the invention, the non-reducing glucans are modified by using glucosyltransferases. Thus the invention comprises, for instance, a method whereby the degree of polymerization of the non-reducing glucans is increased through the use of a phosphorylase in the presence of a glucosyl donor, such as glucose-1-phosphate. It is preferred to use a thermostable phosphorylase at 60-120°C. The thermostable phosphorylase has preferably been obtained from microorganisms belonging to the order of *Thermococcales,* the order of *Thermoproteales*, the genus *Bacillus,* the genus *Thermus* or the genus *Thermotoga*, or a microorganism descending or derived therefrom, more particularly originating from *Pyrococcus furiosus, Pyrococcus woesei, Thermoproteus tenax, Bacillus stearothermophilus, Bacillus caldolyticus, Thermus aquaticus, Thermus thermophilus, Thermus species* ATCC 27737, *Thermotoga maritima,* or a microorganism descending or derived therefrom. Here, too, it holds that according to the invention it is not necessary that an enzyme originating from a particular organism is in fact obtained from this organism; production through a recombinant host is expressly encompassed by the invention.

According to the invention, it is preferred to prepare non-reducing glucans that can be used in food, cosmetics, pharmaceutical or industrial products. For instance, non-reducing glucans are prepared which can be used as fat substitutes or sweetener in food and foodstuffs, or as stabilizer or as filler in food, cosmetics or pharmaceutical products.

The non-reducing glucans obtained can also be derivatized (e.g. chemically, physically or enzymatically), for instance to synthesize detergents. For that purpose, preferably the non-polar molecule tails of the detergent are coupled to (the ends of) the non-reducing glucans.

Formed derivatives, such as detergents, can be hydrolyzed again, for instance through a biocatalytic method or by the use of physicochemical methods. If thereby the α 1,4-bond between the trehalosyl unit and the adjacent glucosyl unit is hydrolyzed (for instance, into a compound of formula XGₙ₋₂TX, wherein X represents a substituent and G, T and n have the aforementioned meanings), this leads to the formation of a monosubstituted trehalosyl unit (for instance, a compound of formula TX). Such a monosubstituted trehalose compound is difficult to synthesize via a conventional route because trehalose is made up of two identical units. The monosubstituted trehalose compound can subsequently be further derivatized, e.g. into a trehalose unit substituted with different groups, having the formula YTX, wherein Y and X are different substituents.

Reducing derivatives, such as detergents, can also be prepared by hydrolysis of the 1,1-bond, either through a biocatalytic method, or with the aid of physicochemical methods.

The invention also comprises a method whereby the trehalose is further derivatized (e.g. physically, chemically or catalytically), for instance to prepare detergents, with the non-polar parts of the detergents being coupled to (the terminal ends of) the trehalose. In that case, too, it holds that reducing detergents on a carbohydrate basis can be prepared by hydrolysis of the 1,1-bond of trehalose, while the hydrolysis of the 1,1-bond can occur through a biocatalytic process or physicochemical methods.

The invention further comprises the non-reducing glucans and products derived therefrom, obtainable or obtained with the above-defined method. The invention also encompasses the use of non-reducing glucans and products derived therefrom as defined above, in food, cosmetics, pharmaceutical or industrial products, in particular as fat substitute or sweetener in foodstuffs, as stabilizer or as filler in foodstuffs, cosmetics and pharmaceutical products, or as detergents.

The invention is also embodied in a thermostable non-reducing glucan-forming enzyme active at a temperature of 60-120°C, preferably 70-80°C. In particular, the invention comprises thermostable NRGFE originating from *Sulfolobus solfataricus* DSM 1617 or a microorganism descending or derived therefrom. The invention also provides an enzyme preparation comprising a thermostable non-reducing glucan-forming enzyme (NRGFE) as defined above, in combination with an enzyme selected from the group consisting of α-amylases, isoamylases, pullulanases, amylopullulanases, amylases, glucosidases, amyloglucosidases, and glucosyltransferases (for instance phosphorylases). Preferably, all enzymes present in such an enzyme preparation are thermostable.

A thermostable enzyme to be used according to the invention can be obtained in the following manner.

### Medium and fermentation conditions

*Sulfolobus solfataricus* DSM-1617 could be cultured in a suitable medium therefor, such as that consisting of the following constituents: yeast extract (2.0 g/l), KH₂PO₄ (3.1 g/l), (NH₄)₂SO₄ (2.5 g/l), MgSO₄.7H₂O (0.2 g/l), CaCl₂.2H₂O (0.25 g/l) and trace elements (0.0018 g/l MnCl₂.4H₂O, 0.0045 g/l Na₂B₄O₇.10H₂O, 0.22.10⁻³ g/l ZnSO₄.7H₂O, 0.05.10⁻³ g/l CuCl₂.2H₂O, 0.03.10⁻³ g/l Na₂MoO₄.2H₂O, 0.03.10⁻³ g/l VOSO₄.2H₂O and 0.01.10⁻³ g/l CoSO₄.7H₂O). With 0.1 M H₂SO₄ the pH was adjusted to 3.5.

Volumes of 5 liters of medium were sterilized in fermentors of a volume of 7 liters (Applikon, Schiedam, Netherlands) by autoclaving for 45 minutes at 120°C and 1.2 bar. Precultures were cultured in 250 ml Erlenmeyer flasks with 70 ml medium (autoclaved for 20 minutes at 120°C and 1.2 bar) at 80°C and 150 rpm in a shaking water bath. The fermentors were inoculated with 280 ml preculture. Growth in the fermentors occurred at 80°C with an aeration of 0.35 liter air/liter medium/minute at a stirring speed of 400 rpm. Growth was monitored by measuring the optical density of the culture at 550 nm and 1 cm path length. At the beginning of the stationary phase with a fermentation period of about 40 hours, the fermentation can be stopped. By centrifugation cells could be separated from the culture liquid.

It is possible to recover NRGFE from cells and from cell-free culture liquid. In doing so, one can use existing technologies for concentration, such as evaporation, ammonium sulfate precipitation, acetone precipitation and membrane filtration, and for purification, such as ion exchange chromatography, gel filtration, isoelectric focussing, hydrophobic interaction chromatography, affinity-chromatography and preparative electrophoresis.

Thus NRGFE could be recovered from cell extracts. To that end, cells were included in 10 mM Tris-HCl buffer (3 ml/g cells wet weight) and subsequently disintegrated at 0°C using ultrasound (disintegrator Soniprep 150, MSE). After centrifugation at 6°C and 35000 g for 60 minutes, the crude cell-free extract was dialyzed against 0.05 M acetate buffer pH 5.5. A crude enzyme preparation was subsequently obtained after 65% ammonium sulfate precipitation followed by acetone precipitation with two volume units of cold acetone. After centrifugation at 6°C and 5000 g for 10 minutes, the precipitated proteins were resuspended in 0.05 M acetate buffer pH 5.5 and dialyzed against this buffer at 6°C.

The thus obtained crude enzyme preparation could be further purified through gel filtration at 6°C with a Hiload 26/60 Superdex column (Pharmacia Biotech AB, Uppsala, Sweden).

The chromatographic conditions were as follows: column volume: 325 ml; elution rate: 1 ml/min; eluent: 0.05 M acetate buffer, 0.15 M NaCl pH 5.5; fraction volume: 5.5 ml; applied: 10 ml crude enzyme preparation.

The following analyses were performed.

### Protein

The elution was monitored by measuring the absorption at 280 nm. Protein concentrations were determined according to the Bradford method with a kit of Bio-Rad and BSA (bovine serum albumin) as standard.

### Oligosaccharide composition of reaction products

The oligosaccharide composition was determined by HPLC. HPLC with a cation exchanger was performed with Benson BCX4 cation exchanger in the calcium form (particle size 15-20 µm, column 250 x 10 mm), with 100 ppm Ca-EDTA as mobile phase, an elution rate of 0.4 ml/min, a column temperature of 85°C and a sample size of 20 µl. The refractive index of the eluted material was determined with a Jasco 830-RI detector at 40°C and registered with an SP4400 Chromjet intregrator of Spectra Physics at a paper rate of 2.5 mm/min. HPLC with an anion exchanger was carried out with a Dionex CarboPac PA1 column (250 x 4 mm) with a CarboPac PA1 guard column. Detection occurred with a Dionex PAD II pulsed amperometric detector of a sensitivity of 1 µC. The elution rate was 1 ml/min, pressure 90 bar and the sample volume 20 µl (150 µl preflush). Used as mobile phase was 0.1 M NaOH (eluent A) with a gradient of 0-0.6 M sodium acetate (eluent B) in the following manner: %B in eluent; 5% (0 min) - 35% (10 min) - 60% (20 min) - 100% (25-27 min) - 5% (28-35 min).

### NRG-forming activity

Substrate: 0.6% soluble starch (Merck) from which the α-1,6 bonds had been removed using isoamylase (Hayashibara Biochem Lab, Japan), dissolved in buffer; enzyme/substrate ratio: 0.5 ml enzyme + 6 ml substrate; temperature: 70°C; reaction time: 18.5 hours; buffer: 0.05 M sodium acetate buffer pH 5.5; analysis: decrease of reducing end groups according to Nelson-Somogyi; activity expressed in units per ml with 1 unit (U) being equal to the decrease of 1 µmol reducing power per minute (amylase activity could be determined in the same manner as the increase of reducing end groups).

Thus one peak with NRG-forming activity obtained eluting between 71 and 138 ml after beginning of the void peak; according to a calibration line drawn up with ribonuclease A (13.7 kDa), chymotrypsinogen A (25 kDa), ovalbumin (43 kDa), albumin (67 kDa), β-amylase (200 kDa) and apoferritin (443 kDa) corresponding with a molecular weight of about 5*10⁴ Da. The active fractions from a number of gel filtrations were collected. After dialysis against 20 mM piperazine pH 5.5 with 0.02% NaN₃, this material was further purified by means of anion exchange chromatography with a Mono Q HR 10/10 FPLC column (Pharmacia Biotech AB, Uppsala, Sweden).

The chromatographic conditions were as follows: column volume: 8 ml; elution rate: 2 ml/min; eluent: 20 mM piperazine pH 5.5; gradient: 0-0.5 M NaCl in eluent in 45 min followed by 0.5-1M NaCl in 25 min and 1 M NaCl for 20 min; fraction volume: 60 ml during application, 2 ml during gradient-elution; volume applied: 867 ml with 38 mg protein.

Assays were carried out as described hereinbefore, with NRG-forming activity being determined with 0.2 ml enzyme and with 4 ml 0.6% maltooligosaccharides having an average degree of polymerization of approx. 6 (DP6) as substrate (according to HPLC containing maltopentaose, maltohexaose and maltoheptaose in the ratio 1:3:1.4).

Thus NRG-forming activity, which appeared present in the run-through with 85% of the protein applied, could be separated from an amylolytic activity, bound to the column material under these conditions, which eluted between 14 and 27 min after start of the gradient.

Material present in the run-through of a number of anion exchange chromatographies performed as described above, was, after dialysis against 0.02 M Tris/HCl pH 7.5 + 0.02% NaN₃, further purified by means of anion exchange chromatography with a Mono Q HR 10/10 column under the following conditions: column volume: 8 ml; elution rate: 1 ml/min; eluent: 0.02 M Tris/HCl pH 7.5; gradient: 0-0.075 M NaCl in eluent in 5 min followed by 0.075-0.225 M NaCl in 60 min, 0.225-0.5 M NaCl in 30 min and 0.5 M NaCl for 25 min; fraction volume: 59 ml during application, 1 ml during gradient elution; volume applied: 1450 ml with 46 mg protein.

Assays were performed as described hereinbefore, with NRG-forming activity being determined with 0.025 ml enzyme and 4 ml 0.6% DP6.

In this way NRG-forming activity eluted between 12 and 35 minutes after start of the gradient. This material was subjected to a dialysis against Milli-Q water. A purified NRGFE useful in the method could thereafter be obtained by multiple separation using the Rotofor isoelectric focusing system of Bio-Rad under the following conditions: applied volume first run: 10 ml dialyzed sample containing 5.5 mg protein (15 mg based on A280 BSA 1 mg/ml=0.68) with 45 ml Milli-Q water, 2.6 ml Ampholine 3.5-10 (Pharmacia Biotech AB, Uppsala, Sweden) and urea in a final concentration of 3 M; power: 12 W, constant; run time: 4 hours; fractions: 20 with a volume of 2.5 ml.

De pH in the fractions rose from 4.03 to 10.78. The NRG-forming activity was determined after dialysis against 0.02 M Tris/HCl pH 7.5 with 0.25 ml enzyme and 4 ml 0.6% DP6. Thus activity was found between fractions with pH 4.66 and 7.22.

With this material a second run was carried out, in which the following variables were used: applied volume second run: 8 ml of fractions from the first run focussing between pH 4.66 and 7.22 containing 1.5 mg (4.7 mg protein based on A280 BSA 1 mg/ml=0.68), with 49 ml Milli-Q water and urea in a final concentration of 3 M; power: 12 W, constant; run time: 3 hours; fractions: 20 with volume of 2.5 ml.

The pH in the fractions rose from 4.05 to 11.87. The NRG-forming activity after dialysis against 0.02 M Tris/HCl pH 7.5 was determined with 0.15 ml enzyme and 4 ml 0.6% DP6 after 2 hours of incubation.

Thus one peak of activity was found with 1.4 mg protein, between fractions with pH 4.71 and 7.01 with the top at pH 5.7.

For the thus obtained purified and dialyzed NRGFE preparation, the following properties were determined.

### pH range

Substrate: 1.2% DP6; buffer: 0.1 M bis-tris-propane + 0.1 M citrate + 0.1 M maleate + 0.02% NaN₃; pH 3-9.5 rising in steps of 0.5; enzyme/substrate ratio: 0.2 ml substrate with 0.2 ml buffer of the desired pH and 0.005 ml NRGFE; reaction time: 17.5 hours; temperature: 70°C; analysis: change in reducing end groups according to Nelson-Somogyi.

Activity was found between pH 4.5 and 9.0 with an optimum at pH 6.0.

### pH stability

Buffer: 0.1 M bis-tris-propane + 0.1 M citrate + 0.1 M maleate + 0.02% NaN₃; pH 3-9.5 with steps of 0.5 pH unit; enzyme: 0.05 ml NRGFE with 0.150 ml buffer of the desired pH; temperature: 21°C; reaction time: 1 hour; analysis: activity was determined as above at pH 6.0 in a mixture with the incubated enzyme mixture, 1.5 ml buffer pH 6.0 and 1.3 ml 1.4% DP6.

It was found that activity of NRGFE had been maintained with incubations performed at pH 5.5-9.5 and had disappeared after incubation at pH 3 and 3.5.

### Temperature range

Substrate: 1.2% DP6 in buffer; buffer: 0.1 M bis-tris-propane + 0.1 M citrate + 0.1 M maleate + 0.02% NaN₃ pH 6.0; enzyme/substrate ratio: 0.15 ml substrate with 0.15 ml NRGFE; reaction time: 0.5 hours; temperature: 50-100°C, in steps of 5°C; analysis: change in reducing end groups.

Activity was observed in the entire temperature range with an optimum at 75°C.

### Temperature stability

Buffer: 0.1 M bis-tris-propane + 0.1 M citrate + 0.1 M maleate + 0.02% NaN₃, pH 6; enzyme: 0.1 ml NRGFE; temperature: 75-100°C, in steps of 5°C; reaction time: 0.5 and 3.5 hours; analysis: activity was determined as above at pH 6.0 with 0.1 ml of the incubated enzyme mixture and 0.1 ml buffer with 1.2% DP6 for 4 hours at 70°C.

In this way it was found that activity had been maintained with incubations performed at 75-90°C; after 0.5 hours at 95°C there still remained 94% of the activity of the NRGFE and after 3.5 hours still 47%. After 0.5 hours at 100°C there was 1% activity left.

Characteristic of the action of the NRGFE is the possibility of forming non-reducing glucans from reducing substrates such as DP6 at temperatures above 60°C.

### Production of non-reducing glucans from DP6

Substrate: 1.2% DP6 in buffer; buffer: 0.1 M bis-tris-propane + 0.1 M citrate + 0.1 M maleate + 0.02% NaN₃, pH 6; enzyme/substrate ratio: 4 ml NRGFE with 4 ml substrate; reaction time: 18 hours; temperature: 70°C; analysis: change in reducing end groups; HPLC.

Thus the amount of reducing end groups was reduced by 68%. The disappearance of reducing power was found to be accompanied by the appearance, on chromatograms which had been obtained after HPLC with anion exchanger, of new peaks with retention times of 6.5, 7.7 and 8.8 minutes next to those of maltopentaose, maltohexaose and maltoheptaose with retention times of 10.4, 11.5 and 12.4 minutes.

These peaks disappeared after incubation of 1.5 ml of the reaction mixture with 1 ml of a solution with 1.05 mg/ml amyloglucosidase (*Aspergillus niger,* 75 u/mg; Merck, Darmstadt, Germany) in 0.05 M maleate buffer pH 5.0 for 21 hours at 37°C. After this treatment it was found that peaks were formed with retention times of 2.2 and 3.0 min, corresponding with those of α,α-trehalose and glucose. From HPLC with cation exchanger a mass ratio of 1:3.4 could be determined for the di- and monosaccharide. After treatment with a solution of amyloglucosidase in combination with 0.032 mg/ml trehalase (pig kidney, 0.68 u/mg; Sigma, St.Louis, USA), solely a peak with a retention time of 3.0 min was found to be formed.

From this it could be concluded that through NRGFE, reducing glucans of the overall formula Gₙ wherein G stands for a glucosyl residue and subscript n for an integer are converted into non-reducing glucans of the overall formula Gₙ₋ ₂T wherein T stands for a trehalosyl unit.

A characteristic property of preparations containing non-reducing glucans is decrease of coloration resulting from the Maillard reaction.

### Maillard reaction

Formation of non-reducing glucans-containing DP6 preparation: 3 ml 2% DP6 in 0.1 M acetate buffer pH 5.7 + 4 mM calcium chloride + 0.04% NaN₃ was incubated with 3 ml NRGFE at 80°C for 18 hours. After incubation the amount of reducing end groups was found to have decreased by 51% compared with similarly treated DP6 that had not been incubated with NRGFE but with 3 ml 0.02 M Tris/HCl.

Reaction: 2 ml of the above liquids + 0.4 ml 10% glycine + 1.6 ml 2.2 mM NaOH; pH 7.1; reaction time: 17 hours; temperature: 95°C; analysis: increase absorption at 450 nm.

In this way, with DP6 an increase of the absorption from 0.005 to 0.164 was established and with the non-reducing-glucans-containing DP6 an increase from 0.008 to 0.045.

The method according to the invention can be carried out with NRGFE produced by *Sulfolobus solfataricus* DSM-1617, but any other organism that produces an NRGFE active above 60°C can also be used. After culturing such organisms in a manner suitable therefor, NRGFE containing sources, such as cells, cell extracts or cell-free culture liquids, can as such be used in the method. Optionally, prior to use, concentration by means of existing technologies, such as centrifugation and filtration, can take place. In cases where components such as hydrolytic activities present in these enzyme sources constitute an impediment to the practice of the method, such an activity can be suppressed, for instance by the use of inhibitors, or the NRGFE can be purified. As described hereinabove for the NRGFE originating from *Sulfolobus solfataricus* DSM-1617, existing techniques can be used for this purpose.

NRG-forming enzyme preparations can be used in immobilized form, so that they can be simply separated from the reaction mixture. This also applies to the enzymes in the combination with which the method according to the invention is carried out.

In enzyme combinations which are used in the method according to the invention, in addition to NRGFE, in principle any enzyme with thermostable properties can be used and preferably enzymes belonging to the class of oxidoreductases, hydrolases, transferases, lyases or isomerases including those enzymes belonging to the subclasses EC 1.1, EC 2.4, EC 2.7. EC 3.1, EC 3.2, EC 4.2, EC 5.1, EC 5.3, EC 5.4 and in particular those forming part of the sub-subclasses EC 1.1.3, EC 2.4.1, EC 2.7.1, EC 3.1.3, EC 3.2.1, EC 4.2.2, EC 5.1.3, EC 5.3.1, EC 5.4.1, EC 5.4.2, with enzymes that are active with starch as substrate being especially of importance.

The composition of the thermostable enzyme combination in the method is determinative of the composition of the products eventually obtained. A person skilled in the art can influence the product composition through a directed choice of the enzymes in combination with which the NRGFE is used. Thus, from starch, by the use of a hydrolase such as an isoamylase (EC 3.2.1.68) or a pullulanase (EC 3.2.1.41), oligosaccharides having a degree of polymerization (DP) greater than 5 can be obtained, which can serve as substrate for the NRGFE used in combination. By the use of the method in this manner, it is possible to obtain a product consisting entirely of non-reducing glucans. When using other enzymes in the combination, specifically hydrolases such as α-amylase (EC 3.2.1.1), β-amylase (EC 3.2.1.2), amyloglucosidase (EC 3.2.1.3), α-glucosidase (EC 3.2.1.20), amylo-pullulanase or transferases such as amylomaltase (EC 2.4.1.25) or cyclomaltodextrin glucanotransferases (EC 2.4.1.19), products with a low DP, such as glucose, can be formed, which cannot be used as substrate by the NRGFE present in the combination. To separate such reaction products from the starting materials, proven technologies such as membrane filtration or batchwise feed/dilution of, respectively, the enzyme combination/substrate and product can be employed. Reactions in two-phase systems can also be employed.

By separating starting materials from reaction products, also an efficiency improvement can arise as a result of the elimination of final product inhibition.

The possibilities of separating undesired reaction products can be improved through specific derivatization of such a component. Thus, by the addition of glucose oxidase (EC 1.1.3.4) glucose can be converted into gluconic acid, which reaction product can be selectively removed through existing technologies.

In the method according to the invention, enzymes can be added which can use the non-reducing glucans formed as substrate, such as hexosyltransferases (EC 2.4.1), in particular the glucosyltransferases such as 4-α-glucanotransferase (EC 2.4.1.25) and trehalose phosphorylase (EC 2.4.1.64) and especially phosphorylase (EC 2.4.1.1) which, depending on reaction conditions, in particular the concentration of inorganic phosphate (Pᵢ), can effect the transfer of a glucosyl unit from or to a non-reducing end of a glucan. In Example 3 this is demonstrated'for phosphorylase from potato.

Research performed by applicant, inter alia, has shown that thermostable phosphorylases occur in the domains of *Archaea, Bacteria* and *Eukarya,* specifically in microorganisms belonging to the order of *Thermococcales,* the order of *Thermoproteales,* the genus *Bacillus,* the genus *Thermus* or the genus *Thermotoga,* viz. *Pyrococcus furiosus, Pyrococcus woesei, Thermoproteus tenax, Bacillus stearothermophilus, Bacillus caldolyticus, Thermus aquaticus, Thermus thermophilus, Thermus species* ATCC 27737 and *Thermotoga maritima.*

Depending on the result contemplated, at a time to be determined by the skilled person, the enzyme combination can be changed in composition, for instance by using immobilized enzymes, while, if necessary, the reaction conditions can be adjusted.

With the method according to the invention, the person skilled in the art, through an appropriate combination of enzymes, can obtain as product non-reducing glucans or products derived therefrom with a degree of polymerization of 2 and higher.

In cases where it is desirable not to add non-reducing glucans beforehand to the products to be prepared, the method according to the invention can be practised *in situ,* an example of a case in point being the preparation of starch containing bakery products where temperatures above 60°C are employed, such as bread-making.

The method according to the invention is carried out at temperatures above 60°C with starch material as substrate. The starch material can be present in aqueous but also in a water-limited environment. An aqueous solution of the starch material, preferably with a starch concentration between 0.1 and 50 percent by weight, is obtained by heating above the gelatinizing temperature of the starch material used or by dissolving pregelatinized starch material in water.

The method according to the invention is carried out with a combination of enzymes, and the person skilled in the art can determine an optimum combination of conditions for use of the desired combination of enzymes on the basis of known data about the individual enzymes, inter alia with regard to pH, temperature and substrate dependency.

When using the NRGFE originating from *Sulfolobus solfataricus* DSM-1617, the method can be carried out at a temperature between 60 and 120°C, preferably between 70 and 80°C. The pH values when using NRGFE from *Sulfolobus solfataricus* DSM-1617 can vary between 4.5 and 9 and preferably between pH 5 and 7. The reaction time is not limited but is preferably between 1 and 100 hours.

The NRG-containing products that are formed with the method according to the invention can be used as such or be stripped of impurities and concentrated with known technologies such as centrifugation, filtration, reverse osmosis and evaporation. If necessary, reducing glucans and other undesired components such as salts can be removed with techniques available therefor. Results described above with HPLC with anion exchanger indicate that ion exchange chromatography can for instance be used to separate the different glucans from each other.

The NRG obtained can be used in methods where starch material is used and where the absence of reducing power is of importance. Applications of starch and starch derivatives are for instance found in the paper, corrugated board, adhesive, detergent, textile and foodstuff industries, in the cosmetic and pharmaceutical industries, and in the oil and gas extraction industry. Such applications are described inter alia by R.J. Alexander in "Maltodextrins: Production, Properties and Applications" (in: Starch hydrolysis products: worldwide technology, production and application. F.W. Schenck, R.E. Hebeda eds., VCH Publishers, New York, 1992, pp. 233-275). Without wishing to be complete, they are methods involving application as fat substitute, sweetener, stabilizer, filler, spray drying additive, adsorbent for flavors, fabric softener, bleach activator, surfactant, emulsifier (detergent), complexing agent, sequestering agent and in coatings and drilling muds. The use of NRG prevents reactions which may involve the reducing end of starch, such as they may, for instance, occur with oxygen, with amino groups of amino acids, peptides and proteins present in products, or with fine chemicals present in the products, such as flavors. NRGs could therefore serve as substitutes of hydrogenated or oxidized starch materials.

The above-mentioned stability is also of advantage when using NRGs instead of starch material as starting material for derivatization reactions which are generally carried out under acid or basic conditions and/or at elevated temperatures. For instance in etherification reactions of starch materials in alkaline environment, the use of NRGs will involve a highly reduced browning.

In the NRG formed through the method according to the invention, an additional non-reducing end has been formed which can be used for further derivatization. This can for instance occur in a continuous process wherein the method according to the invention is carried out in a membrane reactor with an amylase and NRGFE as enzyme combination. The formed NRG with a low DP is thereby removed and can be derivatized in a next compartment. Such a derivatization can be a coupling of non-polar tails to non-reducing ends, so that detergents are formed.

In the NRG obtained by means of the method according to the invention a trehalosyl unit has been introduced through NRGFE.

The NRG formed on the basis of starch by means of the method according to the invention and the substances derived therefrom contain a trehalosyl unit which is characterized by a specific type of bond. The glucan is thus provided with a bond unique to the glucan. In addition to the possibilities arising from the absence of the reducing end, this new bond opens additional possibilities for methods where the NRG is used. Thus this new bond provides the possibility of targetted hydrolysis of NRG and substances derived therefrom. For this purpose, physical, chemical and enzymatic methods can be used, such as the use of NRGFE under conditions where a reversal of the reaction described in this patent specification occurs. Conditions for practising such a method can be determined by a person skilled in the art and may mean that the reaction is carried out in a water-limited environment.

Further, for a person skilled in the art it is possible, by the use of biocatalytic, physical or chemical methods, to hydrolyze the α 1,4-bond between the trehalosyl unit and the adjacent glucosyl unit, which, for substances derived from NRG, can lead to the formation of a monosubstituted trehalosyl unit. Such a hydrolysis could be carried out biocatalytically, for instance by the use of an α-amylase optionally in combination with α-glucosidase or amyloglucosidase.

The invention thus provides the possibility of preparing non-reducing glucans by enzymatic route, which by their specific properties signify an extension or improvement of the possibility of using starch products and which can serve as a basis for further derivatization whereby new products can be obtained and/or on which new methods can be based.

The invention is explained in and by the following examples.

### Example 1

In 8 fermentations of *Sulfolobus solfataricus* DSM-1617 under conditions described hereinbefore, with cells being harvested at an OD₅₅₀ between 0.54 and 0.66, an average cell yield of 1.52 g/l wet weight was obtained. Cells were separated from culture liquid by centrifugation. From the cells, in the manner described hereinbefore, a purified enzyme preparation was obtained with 18.6 µg protein per ml and a specific activity of 2 U/mg. Assays took place as described hereinbefore.

The purified NRGFE containing preparation was used in a method in which potato starch was hydrolyzed with a commercial α-amylase.

2.5 ml of the purified NRGFE preparation was mixed with 2.5 ml of a solution with 0.4 µl Termamyl 120L (Novo Nordisk, Copenhagen, Denmark) per liter 0.1 M acetate buffer pH 5.7 + 4 mM CaCl₂ + 0.04 % NaN₃ and 5 ml 1.2% native potato starch (85% dry solids; AVEBE, Foxhol, Netherlands) in the same buffer. The reaction mixture was incubated at 80°C and after 24 and 48 hours 2 ml of sample was taken which, to stop the reaction, was set in ice water and was adjusted to pH 8.0 with 50 µl 1M Tris/HCl pH 8.5. In the samples the number of reducing end groups was determined and, by means of HPLC with cation exchanger, the content of products with a DP1-7, this as a measure for the decomposition of the starch by the α-amylase. The reducing power, expressed as dextrose equivalent (DE), was 6.9 after 24 hours and 11.1 after 48 hours. The content of DP1-7 was, respectively, 3.1 and 3.5 g/l, corresponding with 62 and 68% breakdown. HPLC with anion exchanger further showed the presence of products with retention times corresponding with those of non-reducing glucans. For comparison, the same reaction was carried out with 2.5 ml buffer without NRGFE. After 24 hours the DE in this reaction mixture was 11.2 and after 48 hours 18.5. The content of DP1-7 was, respectively, 2.5 and 3.3 g/l, corresponding with 51 and 65% decomposition of the native starch. HPLC with anion exchanger samples gave no indication of the presence of non-reducing glucans in this sample. So, given an equal decomposition, by the use of an enzyme combination with NRGFE the reducing power has remained considerably lower.

### Example 2

The purified NRGFE preparation from Example 1 was used in combination with a commercial pullulanase.

A solution with 0.72 % soluble starch (90% dry solids; Merck, Darmstadt, Germany) in 0.1 M acetate buffer pH 5.7 + 4 mM CaCl₂ + 0.04 % NaN₃ was gelatinized by heating at 100°C for 15 minutes. After cooling to 60°C there was added to 5 ml of this solution 0.4 ml of the purified NRGFE preparation and 0.6 ml of a solution with 40 µl Promozyme 200L (Novo Nordisk, Copenhagen, Denmark) per liter in buffer. A similar incubation was performed without NRGFE. After 16.5 hours incubation of the reaction mixtures at 60°C the DE was determined. Without NRGFE it was found to have increased from 0.91 to 0.98 by the action of Promozyme. Combination of Promozyme with NRGFE resulted in a DE of 0.87, and the presence in this sample of products with a retention time corresponding with that of non-reducing glucans could be demonstrated with the aid of HPLC with anion exchanger.

### Example 3

A reaction mixture consisting of 3 ml 0.1 M acetate buffer + 4 mM CaCl₂ + 0.04% NaN₃ with 2% DP6 and 3 ml of the purified NRGFE preparation was incubated at 80°C for 18 hours. The number of reducing ends in this reaction mixture was found to have decreased by 51% compared with an incubation carried out without NRGFE. Both products, DP6 and NRG-containing DP6, were used as substrate for phosphorylase recovered from potato according to known methods. Reaction mixtures consisting of 0.375 ml of the above mixtures, with an activity of 2 units per ml, 2.5 ml phosphorylase (2 units per ml) and 5 ml 3% α-glucose-1-phosphate (G-1-P) in 0.05 M bis-tris-propane pH 7 were incubated at 35°C for 24 hours. The decrease in G-1-P calculated from the amount of inorganic phosphate formed, determined with the method of Chen, was found to be the same in both reaction mixtures (40%). The amount of polymeric material formed, determined with the anthrone-sulfuric acid method, after dialysis of 2 ml of the reaction mixtures against 0.1 M bis-tris-propane pH 7, was also found to be the same. After determination of the reducing power of the polymeric material from the two reaction mixtures, a DP of 50 could be calculated for the polymer from the reaction mixture with DP6, and a DP of 75 for the reaction mixture with NRG-containing DP6. To demonstrate that NRG had been used as primer for the polymer formed, the solutions obtained upon dialysis were treated with amyloglucosidase by incubating 0.2 ml of the dialyzed solutions with 0.4 ml amyloglucosidase (1 mg/ml; *Aspergillus niger,* 75 u/mg; Merck, Darmstadt, Germany) in 0.1 M acetate buffer pH 4.1 at 35°C for 15 hours. After incubation, with HPLC with anion exchanger no trehalose was demonstrable in polymer obtained with DP6, but it *was* in that obtained with NRG-containing DP6 as primer.

## Claims

1. A method for conversion of a starch material, **characterized in that** the conversion takes place utilizing a non-reducing glucan-forming enzyme activity (NRGFE), catalysing the reaction Gₙ → Gₙ₋₂T, which NRGFE is active above 60°C and originates from a thermophilic archaeon of the genus *Sulfolobus.*

2. A method according to claim 1, **characterized in that** the conversion takes place at 60-120°C, preferably 70-80°C.

3. A method according to claim 1 or 2, **characterized in that** the conversion takes place at pH 4-9, preferably pH 5-7.

4. A method according to any one of claims 1-3, **characterized in that** the NRGFE originates from *Sulfolobus solfataricus* DSM 1617 or a micro-organism descending or derived therefrom.

5. A method according to any one of claims 1-4, **characterized in that** as starch material a native starch is used.

6. A method according to any one of claims 1-5, **characterized in that** the starch material is converted under simultaneous formation of non-reducing glucans Gₙ₋₂T.

7. A method according to claim 6, **characterized in that** the conversion is carried out with the aid of glucanases which are active above 60°C, in particular α-amylases, isoamylases, pullulanases, amylopullulanases or combinations thereof.

8. A method according to any one of claims 1-7, **characterized in that** by the use of glucanases, such as amylases, glucosidases, amyloglucosidases or combinations thereof, trehalose is formed from the non-reducing glucans Gₙ₋₂T.

9. A method according to any one of claims 1-8, **characterized in that** the non-reducing glucans Gₙ₋₂T are modified using glucosyltransferases.

10. A method according to claim 9, **characterized in that** by the use of phosphorylase in the presence of a glucosyl donor, such as glucose-1-phosphate, the degree of polymerization of the non-reducing glucans Gₙ₋₂T is raised.

11. A method according to claim 10, **characterized in that** a phosphorylase which is active above 60°C is used at 60-120°C.

12. A method according to claim 11, **characterized in that** said phosphorylase has been obtained from micro-organisms belonging to the order of *Thermococcales*, the order of *Thermoproteales*,, the genus *Bacillus*, the genus *Thermus* or the genus *Thermotoga*, or a microorganism descending or derived therefrom.

13. A method according to claim 12, **characterized in that** said phosphorylase originates from *Pyrococcus furiosus, Pyrococcus woesei, Thermoproteus tenax, Bacillus stearothermophilus, Bacillus caldolyticus, Thermos aquaticus, Thermus thermophilus, Thermus species* ATCC 27737, *Thermotoga maritima*, or a micro-organism descending or derived therefrom.

14. A method according to any one of claims 1-13, **characterized in that** non-reducing glucans Gₙ₋₂T are prepared which can be used in food, cosmetics, pharmaceutical or industrial products.

15. A method according to claim 14, **characterized in that** non-reducing glucans Gₙ₋ ₂T are prepared, which can be used as fat substitutes or as sweetener in food and foodstuffs, or as stabilizer or as filler in food, cosmetics or pharmaceutical products.

16. A method according to any one of claims 1-15, **characterized in that** the non-reducing glucans Gₙ₋₂T are derivatized.

17. A method according to claim 16, **characterized in that** from the non-reducing glucans Gₙ₋₂T, detergents are synthesized.

18. A method according to claim 17, **characterized in that** of the detergent, the non-polar molecule tails are coupled to the non-reducing glucans Gₙ₋₂T.

19. A method according to any one of claims 16-18, **characterized in that** derivatized non-reducing glucans are hydrolyzed.

20. A method according to claim 19, **characterized in that** of the derivatized non-reducing glucans, the a 1,4-bond between the trehalosyl unit and the adjacent glucosyl unit is hydrolyzed, whereby a monosubstituted trehalose is obtained.

21. A method according to any one of claims 1-19, **characterized in that** reducing derivatives, such as detergents, are prepared through hydrolysis of the 1,1-bond.

22. A method according to claim 21, **characterized in that** the hydrolysis of the 1,1-bond is effected biocatalytically or with the aid of physicochemical methods.

23. A method according to claim 8 or 20, **characterized in that** the optionally substituted trehalose is derivatized.

24. A method according to claim 23, **characterized in that** on the basis of the trehalose, detergents are prepared.

25. A method according to claim 24, **characterized in that** of the detergent, the non-polar parts are coupled to the trehalose.

26. A method according to claim 25, **characterized in that** reducing detergents on a carbohydrate basis are prepared by means of hydrolysis of the 1,1-bond of trehalose.

27. A method according to claim 26, **characterized in that** the hydrolysis of the 1,1-bond occurs through a biocatalytic process or through physicochemical methods.

28. Non-reducing glucan-forming enzyme catalysing the reaction Gₙ → Gₙ₋₂T which is active at a temperature of 60-120°C, preferably 70-80°C, and originates from a thermophilic archaeon of the genus *Sulfolobus*.

29. Enzyme according to claim 28, originating from *Sulfolobus solfataricus* DSM 1617 or a micro-organism descending or derived therefrom.

30. Enzyme preparation comprising a non-reducing glucan-forming enzyme (NRGFE) as defined in claim 28 or 29, in combination with an enzyme selected from the group consisting of a-amylases, isoamylases, pullulanases, amylopullulanases, amylases, glucosidases, amyloglucosidases, and glucosyltransferases, such as phosphorylases.

31. Enzyme preparation according to claim 30, wherein each of the enzymes present therein is active above 60°C.

## Patentansprüche

1. Verfahren zur Umwandlung von Stärkematerial, **dadurch gekennzeichnet, dass** die Umwandlung unter Verwendung einer nicht-reduzierenden Glucan-bildenden Enzymaktivität (NRGFE) erfolgt, die die Reaktion Gₙ -> Gₙ₋₂T katalysiert, wobei das NRGFE oberhalb von 60 °C wirksam ist und von einem thermophilen Archaeon des Genus *Sulfolobus* abstammt.

2. Verfahren nach Anspruch 1, **dadurch gekennzeichnet, dass** die Umwandlung bei 60 bis 120 °C, vorzugsweise 70 bis 80 °C stattfindet.

3. Verfahren nach Anspruch 1 oder 2, **dadurch gekennzeichnet, dass** die Umwandlung bei einem pH-Wert von 4 bis 9, vorzugsweise einen pH-Wert von 5 bis 7 stattfindet.

4. Verfahren nach einem der Ansprüche 1 bis 3, **dadurch gekennzeichnet, dass** das NRGFE sich von *Sulfolobus solfataricus* DSM 1617 oder einem Mikroorganismus stammt, der davon abstammt oder sich davon ableitet.

5. Verfahren nach einem der Ansprüche 1 bis 4, **dadurch gekennzeichnet, dass** als Stärkematerial eine natürliche Stärke verwendet wird.

6. Verfahren nach einem der Ansprüche 1 bis 5, **dadurch gekennzeichnet, dass** das Stärkematerial unter gleichzeitiger Bildung von nicht-reduzierendenden Glucanen Gₙ₋₂T umgewandelt wird.

7. Verfahren nach Anspruch 6, **dadurch gekennzeichnet, dass** die Umwandlung mit Hilfe von Glucanasen durchgeführt wird, die oberhalb von 60 °C wirksam sind, insbesondere α-Amylasen, Isoamylasen, Pullulanasen, Amylopullulanasen oder Kombinationen derselben.

8. Verfahren nach einem der Ansprüche 1 bis 7, **dadurch gekennzeichnet, dass** durch die Verwendung von Glucanasen wie beispielsweise Amylasen, Glucosidasen, Amyloglucosidasen oder Kombinationen derselben aus den nicht-reduzierenden Glucanen Gₙ₋₂T Trehalose gebildet wird.

9. Verfahren nach einem der Ansprüche 1 bis 8, **dadurch gekennzeichnet, dass** die nicht-reduzierenden Glucane Gₙ₋₂T unter Verwendung von Glucosyltransferasen modifiziert werden.

10. Verfahren nach Anspruch 9, **dadurch gekennzeichnet, dass** durch die Verwendung von Phosphorylase in Gegenwart eines Glucosyldonators wie beispielsweise Glucose-1-phosphat der Polymerisationsgrad der nicht-reduzierenden Glucane Gₙ₋₂T erhöht wird.

11. Verfahren nach Anspruch 10, **dadurch gekennzeichnet, dass** eine Phosphorylase, die oberhalb von 60 °C wirksam ist, bei 60 bis 120 °C verwendet wird.

12. Verfahren nach Anspruch 11, **dadurch gekennzeichnet, dass** die Phosphorylase aus Mikroorganismen erhalten worden ist, die zur Ordnung der *Thermococcalen*, der Ordnung der *Thermoprotealen,* dem Genus *Bacillus,* dem Genus *Thermus* oder dem Genus *Thermotoga* oder einem davon abstammenden oder abgeleiteten Mikroorganismus gehören.

13. Verfahren nach Anspruch 12, **dadurch gekennzeichnet, dass** die Phosphorylase von *Pyrococcus furiosus, Pyrococcus woesei, Thermoprokeus tenax, Bacillus stearothermophilus, Bacillus caldolyticus, Thermus aquaticus, Thermus thermophilus, Thermus Spezies* ATCC 27737, *Thermotoga maritima* oder einem davon abstammenden oder abgeleiteten Mikroorganismus stammt.

14. Verfahren nach einem der Ansprüche 1 bis 13, **dadurch gekennzeichnet, dass** nicht-reduzierende Glucane Gₙ₋₂T hergestellt werden, die in Lebensmitteln, Kosmetika, pharmazeutischen oder industriellen Produkten verwendet werden können.

15. Verfahren nach Anspruch 14, **dadurch gekennzeichnet, dass** nicht-reduzierende Glucane Gₙ₋₂T hergestellt werden, die als Fettersatzstoffe oder als Süßungsmittel in Lebensmitteln und Lebensmittelprodukten oder als Stabilisator oder als Füllstoff in Lebensmitteln, Kosmetika oder pharmazeutischen Produkten verwendet werden können.

16. Verfahren nach einem der Ansprüche 1 bis 15, **dadurch gekennzeichnet, dass** die nicht-reduzierende Glucane Gₙ₋₂T derivatisiert werden.

17. Verfahren nach Anspruch 16, **dadurch gekennzeichnet, dass** aus den nicht-reduzierenden Glucanen Gₙ₋₂T Detergenzien synthetisiert werden.

18. Verfahren nach Anspruch 17, **dadurch gekennzeichnet, dass** die unpolaren Molekülschwänze des Detergenz an die nicht-reduzierenden Glucane Gₙ₋₂T gekoppelt werden.

19. Verfahren nach einem der Ansprüche 16 bis 18, **dadurch gekennzeichnet, dass** die derivatisierten nicht-reduzierenden Glucane hydrolysiert werden.

20. Verfahren nach Anspruch 19, **dadurch gekennzeichnet, dass** von den derivatisierten nicht-reduzierenden Glucanen eine 1,4-Bindung zwischen der Trehalosyleinheit und der angrenzenden Glucosyleinheit hydrolysiert wird, wodurch eine monosubstituierte Trehalose erhalten wird.

21. Verfahren nach einem der Ansprüche 1 bis 19, **dadurch gekennzeichnet, dass** durch Hydrolyse der 1,1-Bindung reduzierende Derivate wie beispielsweise Detergenzien hergestellt werden.

22. Verfahren nach Anspruch 21, **dadurch gekennzeichnet, dass** die Hydrolyse der 1,1-Bindung biokatalytisch oder mit Hilfe von physikochemischen Verfahren bewirkt wird.

23. Verfahren nach Anspruch 8 oder 20, **dadurch gekennzeichnet, dass** die gegebenenfalls substituierte Trehalose derivatisiert wird.

24. Verfahren nach Anspruch 23, **dadurch gekennzeichnet, dass** auf Basis der Trehalose Detergenzien hergestellt werden.

25. Verfahren nach Anspruch 24, **dadurch gekennzeichnet, dass** die unpolaren Teile des Detergenz an die Trehalose gekoppelt werden.

26. Verfahren nach Anspruch 25, **dadurch gekennzeichnet, dass** mittels Hydrolyse der 1,1-Bindung von Trehalose reduzierende Detergenzien auf einer Kohlenhydratbasis hergestellt werden.

27. Verfahren nach Anspruch 26, **dadurch gekennzeichnet, dass** die Hydrolyse der 1,1-Bindung durch einen biokatalytischen Vorgang oder durch physikochemische Verfahren erfolgt.

28. Nicht-reduzierendes Glucan-bildendes Enzym, das die Reaktion Gₙ -> Gₙ₋₂T katalysiert, bei einer Temperatur von 60 bis 120 °C, vorzugsweise 70 bis 80 °C wirksam ist und von einem thermophilen Arachaeon des Genus *Sulfolobus* stammt.

29. Enzym nach Anspruch 28, das von *Sulfolobus solfataricus* DSM 1617 oder einem davon abstammenden oder abgeleiteten Mikroorganismus stammt.

30. Enzymzubereitung, die ein nicht-reduzierendes Glucan-bildendes Enzym (NRGFE) gemäß Anspruch 28 oder 29 in Kombination mit einem Enzymumfasst, das aus der Gruppe bestehend aus α-Amylasen, Isoamylasen, Pullulanasen, Amylopullulanasen, Amylasen, Glucosidasen, Amyloglucosidasen und Glucosyltransferasen wie beispielsweise Phosphorylasen ausgewählt ist.

31. Enzymzubereitung nach Anspruch 30, bei der jedes der darin vorhandenen Enzyme oberhalb von 60 °C wirksam ist.

## Revendications

1. Procédé de conversion d'un matériel d'amidon, **caractérisé en ce que** la conversion a lieu en utilisant une activité enzyme de formation de glucanes non-réducteurs (NRGFE), qui catalyse la réaction Gₙ→Gₙ₋₂T, laquelle NRGFE est active au-dessus de 60°C et provient d'un archaeon thermophile du genre *Sulfolobus*.

2. Procédé selon la revendication 1, **caractérisé en ce que** la conversion a lieu entre 60 et 120°C, de préférence entre 70 et 80°C.

3. Procédé selon la revendication 1 ou 2, **caractérisé en ce que** la conversion a lieu à pH 4-9, de préférence pH 5-7.

4. Procédé selon l'une quelconque des revendications 1 à 3, **caractérisé en ce que** la NRGFE provient de DSM 1617 de *Sulfolobus solfataricus* ou d'un micro-organisme descendant ou dérivé de celui-ci.

5. Procédé selon l'une quelconque des revendications 1 à 4, **caractérisé en ce que,** en tant que matériel d'amidon, un amidon natif est utilisé.

6. Procédé selon l'une quelconque des revendications 1 à 5, **caractérisé en ce que** le matéfleli d'amidon est converti pendant la formation simultanée de glucanes non-réducteurs Gₙ₋₂T.

7. Procédé selon la revendication 6, **caractérisé en ce que** la conversion est effectuée à l'aide de glucanases qui sont actives au-dessus de 60°C, en particulier des α-amylases, isoamylases, pullulanases, amylopullulanases ou des combinaisons de celles-ci.

8. Procédé selon l'une quelconque des revendications 1 à 7, **caractérisé en ce que**, du fait de l'utilisation de glucanases, telles que des amylases, glucosidases, amyloglucosidases ou combinaisons de celles-ci, un tréhalose est formé à partir des glucanes non-réducteurs Gₙ₋₂T.

9. Procédé selon l'une quelconque des revendications 1 à 8, **caractérisé en ce que** les glucanes non-réducteurs Gₙ₋₂T sont modifiés en utilisant des glucosyltransférases.

10. Procédé selon la revendication 9, **caractérisé en ce que,** du fait de l'utilisation d'une phosphorylase en présence d'un donneur de glucosyle, tel que glucose-1-phosphate, le degré de polymérisation des glucanes non-réducteurs Gₙ₋₂T est augmenté.

11. Procédé selon la revendication 10, **caractérisé en ce qu'**une phosphorylase qui est active au-dessus de 60°C est utilisée entre 60 et 120°C.

12. Procédé selon la revendication 11, **caractérisé en ce que** ladite phosphorylase a été obtenue à partir de micro-organismes appartenant à l'ordre de *Thermococcales*, l'ordre de *Thermoproteales*, le genre *Bacillus*, le genre *Thermus* ou le genre *Thermotoga*, ou d'un micro-organisme descendant ou dérivé de ceux-ci.

13. Procédé selon la revendication 12, **caractérisé en ce que** ladite phosphorylase provient de *Pyrococcus furiosus, Pyrococcus woesei, Thermo* *proteus tenax, Bacillus stearothermophilus, Bacillus caldolyticus, Thermus aquaticus, Thermus termophilus, espèce Thermus* ATCC 27737, *Thermotoga maritima*, ou d'un micro-organisme descendant ou dérivé de ceux-ci.

14. Procédé selon l'une quelconque des revendications 1 à 13, **caractérisé en ce que** des glucanes non-réducteurs Gₙ₋₂T sont préparés qui peuvent être utilisés dans des produits alimentaires, cosmétiques, pharmaceutiques ou industriels.

15. Procédé selon la revendication 14, **caractérisé en ce que** des glucanes non-réducteurs Gₙ₋₂T sont préparés, qui peuvent être utilisés en tant que substituts de graisse ou en tant qu'édulcorant dans un aliment et des denrées alimentaires, ou en tant que stabilisant ou charge dans des produits alimentaires, cosmétiques ou pharmaceutiques.

16. Procédé selon l'une quelconque des revendications 1 à 15, **caractérisé en ce que** les glucanes non-réducteurs Gₙ₋₂T sont modifiés.

17. Procédé selon la revendication 16, **caractérisé en ce que,** à partir des glucanes non-réducteurs Gₙ₋₂T, des détergents sont synthétisés.

18. Procédé selon la revendication 17, **caractérisé en ce que** dans le détergent, les queues de molécules non-polaires sont couplées aux glucanes non-réducteurs Gₙ₋₂T.

19. Procédé selon l'une quelconque des revendications 16 à 18, **caractérisé en ce que** des glucanes non-réducteurs modifiés sont hydrolysés.

20. Procédé selon la revendication 19, **caractérisé en ce que** dans les glucanes non-réducteurs modifiés, la liaison 1,4 entre l'unité tréhalosyle et l'unité glucosyle adjacente est hydrolysée, de sorte qu'un tréhalose mono-substitué est obtenu.

21. Procédé selon l'une quelconque des revendications 1 à 19, **caractérisé en ce que** des dérivés réducteurs, tels que des détergents, sont préparés par hydrolyse de la liaison 1,1.

22. Procédé selon la revendication 21, **caractérisé en ce que** l'hydrolyse de la liaison 1,1 est effectuée de manière biocatalytique ou à l'aide de procédés physicochimiques.

23. Procédé selon la revendication 8 ou 20, **caractérisé en ce que** le tréhalose substitué de manière facultative est modifié.

24. Procédé selon la revendication 23, **caractérisé en ce que,** sur la base du tréhalose, des détergents sont préparés.

25. Procédé selon la revendication 24, **caractérisé en ce que** dans le détergent, les parties non-polaires sont couplées au tréhalose.

26. Procédé selon la revendication 25, **caractérisé en ce que** des détergents réducteurs sur une base hydrate de carbone sont préparés par l'intermédiaire d'une hydrolyse de la liaison 1,1 du tréhalose.

27. Procédé selon la revendication 26, **caractérisé en ce que** l'hydrolyse de la liaison 1,1 a lieu par un procédé biocatalytique ou par des procédés physicochimiques.

28. Enzyme de formation de glucanes non-réducteurs catalysant la réaction Gₙ→Gₙ₋₂T qui est active à une température de 60 à 120°C, de préférence 70 à 80°C, et qui provient d'un archaeon thermophile du genre *Sulfolobus*.

29. Enzyme selon la revendication 28, provenant de DSM 1617 de *Sulfolobus solfataricus* ou d'un micro-organisme descendant ou dérivé de celui-ci.

30. Préparation enzymatique comportant une enzyme de formation de glucanes non-réducteurs (NRGFE) selon la revendication 28 ou 29, en combinaison avec une enzyme sélectionnée parmi le groupe constitué de a-amylases, isoamylases, pullulanases, amylopullulanases, amylases, glucosidases, amyloglucosidases, et glucosyltransférases, telles que des phosphorylases.

31. Préparation enzymatique selon la revendication 30, dans laquelle chacune des enzymes présentes ici est active au-dessus de 60°C.
